# EUROPEAN PATENT APPLICATION

(11) **EP 3 400 866 A1**
(43) Date of publication of application: **14.11.2018**
(21) Application number: 18171468.4
(22) Date of filing: 09.05.2018
(51) Int. Cl.: A61B 5/00, A61B 5/024, A61B 5/021, A61B 5/1455, F04B 43/04, F04B 53/10, H01L 41/09, F04B 45/047

(54) **WEARABLE DEVICE**

(30) Priority: 12.05.2017 TW 106115873
(71) Applicant: Microjet Technology Co., Ltd, Hsin Chu (TW)
(72) Inventor: Chen, Shih-Chang, Hsinchu (TW); Mo, Li-Pang, Hsinchu (TW); Huang, Chi-Feng, Hsinchu (TW); Tsai, Chang-Yen, Hsinchu (TW)
(74) Representative: Uexküll & Stolberg

(57) **Abstract**

A wearable device (2) comprises a band structure (20), a main body (21), a micro air pump (22), an airbag (23), a driving control module (25), a sensor (24) and an optical sensor (28). The micro pump (22) is disposed within the receiving space. The sensor (24) is disposed on the driving control module (25) and connected with the micro air pump (22). The driving control module (25) control the micro air pump (22) to operate, making gas transported from the micro air pump (22) to the airbag (23) so that the airbag (23) is inflated and expended to be fixed on a specific part of the user. The user is able to select the sensor (24) to detect the physiological data, by which a precise detection is achieved. The user is also able to select the optical sensor (28) to detect the physiological data, by which a rapid detection is achieved, and the physiological data is sent to the driving control module (25) to be recorded.

## Description

### FIELD OF THE INVENTION

The present disclosure relates to a wearable device, and more particularly to a wearable device using a piezoelectrically actuated micro air pump to sense physiological data of the user.

### BACKGROUND OF THE INVENTION

In the modern society, rapidity is emphasized and personal pressure is increasing day by day. Under such background, the consciousness of pursuing personal health has gradually risen and developed, in which people begin to have a desire to constantly monitor or examine their own health conditions. In general, traditional way to measure data of human physiological and health information is mainly implemented through a fixed sphygmomanometer or bulky testing instruments. Such testing instruments usually include the components such as motor-type air pumps, airbags, sensors, deflation valves and batteries. However, the motor-type air pumps are easily worn down by the friction, and these components are bulky after being assembled, which causes unsuitability for regular use. If a motor type air pump in smaller size is used instead, the speed of being worn down would be faster and would consume more energy.

Considering the convenience for people to monitor their health regularly and the portability of the monitor device, wearable health-monitoring devices are continuously developed to the market. The common wearable health-monitoring devices usually adopt optical detection to detect the data. However, optical detection is not precise enough as it usually leads to error values so that reliable data cannot be obtained effectively. Under this circumstance, users cannot obtain the precise physiological data and inaccuracy of judgment may occur. On the contrast, if a detecting manner which is more precise is adopted, it takes longer time despite the error value is decreased and correctness of the physiological data may is increased.

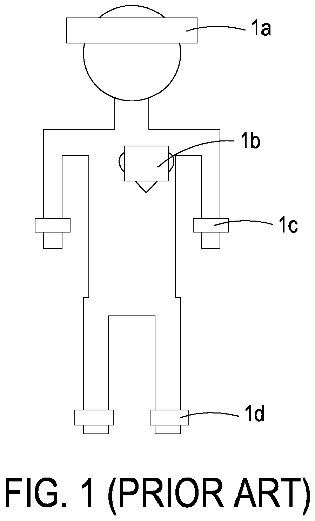

Please refer to FIG. 1. In general, to detect physiological data of a user, a head part 1a, a heart part 1b, a wrist part 1c or an ankle part 1d of the user is usually chosen to be the monitored position as shown in FIG. 1. Those parts are the positions that the pulse, the blood pressure and the heart beat are easiest to be sensed, so that sensing the physiological data at those positions can obtain the physiology and health information rapidly and effectively. However, as mentioned in the above paragraphs, the wearable health-monitoring device to which optical detection is applied fails to obtain reliable data because optical detection is insufficiently precise. Furthermore, since conventional sphygmomanometers or other instruments which are more reliable than optical detection are often bulky, the targets of being light, thin and portable cannot be achieved, and it also costs much more time when using the convention instruments to detect. If the users want to obtain the physiological data immediately, the conventional instruments are inconvenient in practical utilization.

Therefore, there is actually an urgent need to develop a wearable device, which is miniaturized, small, portable, power-saving, and achieves fast and high-precision detection.

### SUMMARY OF THE INVENTION

The main purpose of the present disclosure is to provide a wearable device sensing physiological data with a piezoelectrically actuated micro air pump. By using the piezoelectric actuated micro air pump to transport gas into the airbag, the airbag is inflated and expended, and then the physiological data of user is sensed through a sensor disposed in opposition to the airbag. The present disclosure solves the drawbacks of the prior art such as large volumes, difficulty to be miniaturized, and being not portable.... etc. In addition, the present disclosure solves the problems that it takes long time to sense the physiological data and the conventional health monitoring device using the optical detection technique is not sufficiently precise.

In accordance with an aspect of the present disclosure, there is provided a wearable device. The wearable device comprises a band structure, a main body, a micro air pump, an airbag, a driving control module, a sensor and an optical sensor. The band structure has an outer surface and an inner surface. The main body is disposed on the outer surface of the band structure and connected thereto. The main body has a receiving space. The micro air pump is disposed within the receiving space of the main body. The airbag is disposed on the inner surface of the band structure and opposing to the micro air pump. The airbag is communicated with the micro air pump. The driving control module is disposed within the receiving space of the main body. The sensor is disposed on the driving control module and connected with the micro air pump. The optical sensor is disposed on the inner surface of the band structure and electrically connected with the driving control module. The driving control module drives the micro air pump to operate, thereby transporting gas into the airbag from the micro air pump. The airbag is inflated and expended to be fixed on a specific part of the user. The user is able to select the sensor to detect the physiological data of the user and the physiological data is sent to the driving control module to be recorded, by which a precise detection is achieved. The user is also able to select the optical sensor to detect the physiological data of the user and the physiological data is sent to the driving control module to be recorded, by which a rapid detection is achieved.

The above contents of the present disclosure will become more readily apparent to those ordinarily skilled in the art after reviewing the following detailed description and accompanying drawings, in which:

### BRIEF DESCRIPTION OF THE DRAWINGS

FIG. 1 schematically illustrates positions of measuring physiological data of a user of prior art;
FIG. 2 schematically illustrates the entire structure of a wearable device according to an embodiment of the present disclosure;
FIG. 3A schematically illustrates the exploded view of a micro air pump of the wearable device shown in FIG. 2;
FIG. 3B schematically illustrates the assembled structure of the micro air pump shown in FIG. 3A;
FIG. 4A schematically illustrates a side view of the wearable device shown in FIG. 2.
FIG. 4B schematically illustrates an inflated state of the airbag of a wearable device according to an embodiment of the present disclosure;
FIG. 5 schematically illustrates a wearable device according to an embodiment of the present disclosure worn on a wrist of a user; and
FIG. 6 schematically illustrates the configuration of a wearable device according to an embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this invention are presented herein for purpose of illustration and description only. It is not intended to be exhaustive or to be limited to the precise form disclosed.

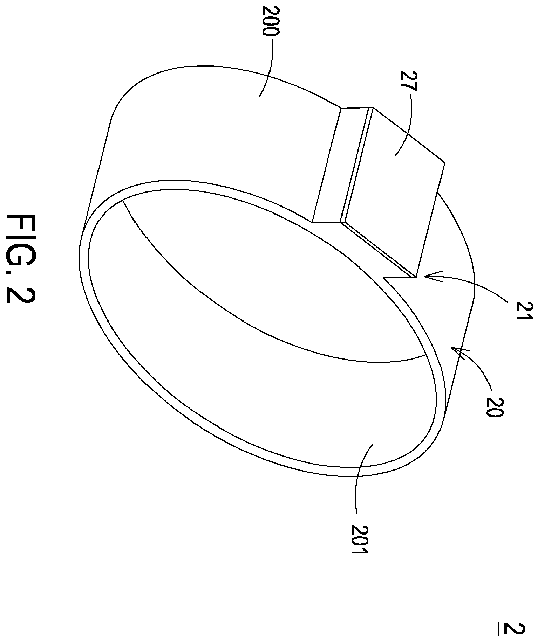

Please refer to FIG. 2 to FIG. 6. A wearable device 2 of the present disclosure is provided to a user to be worn on a specific part of the user, in which the specific part can be the head part 1a, the heart part 1b, the wrist part 1c, the ankle part Id (as shown in FIG. 1) or any other body part. In this embodiment, the wearable device 2 comprises a band structure 20, a main body 21, a micro air pump 22, an airbag 23, a sensor 24, a driving control module 25, a transmission module 26 and an optical sensor 28. The band structure 20 has an outer surface 200 and an inner surface 201. The outer surface 200 is opposite to the inner surface 201. The main body 21 is disposed on the outer surface 200 of the band structure 20 and connected thereto. The main body 21 has a receiving space (not shown) inside for accommodating the micro air pump 22, the driving control module 25 and the transmission module 26. The airbag 23 is disposed on the inner surface 201 of the band structure 20 and opposing to the micro air pump 22. The airbag 23 is communicated with the micro air pump 22. The driving control module 25 is configured to control the operation of the micro air pump 22 for transporting gas through the micro air pump 22 into the airbag 23. As a result, the airbag 23 is inflated and expended to press upon the specific part of the user where the user wears the wearable device 2 on. The sensor 24 is disposed on the driving control module 25 and connected with an outlet end (not shown) of the micro air pump 22 for sensing the physiological data of the user. The physiological data can be transmitted to the driving control module 25 for being recorded. The optical sensor 28 is disposed on the inner surface 201 of the band structure 20 and electrically connected with the driving control module 25. The optical sensor 28 further has a light emitter 281 and a light receiver 282, a sensing light beam is emitted by the light emitter 281 to sense the physiological data, and then the sensing light beam is received by the light receiver 282. The optical sensor 28 can transmit the physiological data to the driving control module 25 for recording.

Please refer to FIG. 2. In this embodiment, the band structure 20 of the wearable device 2 can be a ring structure made of soft or hard materials (e.g. silicone, plastic, webbing, towel, leather, metal or other relevant materials that can be used). The band structure 20 is mainly for being sleeved on the specific part of the user, such as the wrist or the ankle, but not limited thereto. The total length of the band structure 20 is not limited, as in some embodiments, the band structure 20 may be webbing or a towel band for being fixed on the head part of the user. In some other embodiments, the band structure 20 can also be an auxiliary fixing band made of plastic for surrounding the chest area of the user for monitoring the physiological information of the heart part of the user. The connection manners of the two ends of the band structure 20 includes but not limited to hook-and-loop fastener (Velcro), buckle with a convex part and a concave part correspondingly jointed, and buckle ring which is commonly used for watch bands. Moreover, the band structure 20 may be integrally formed in one piece. The connection manners can be varied to meet the practical demands, but not limited herein.

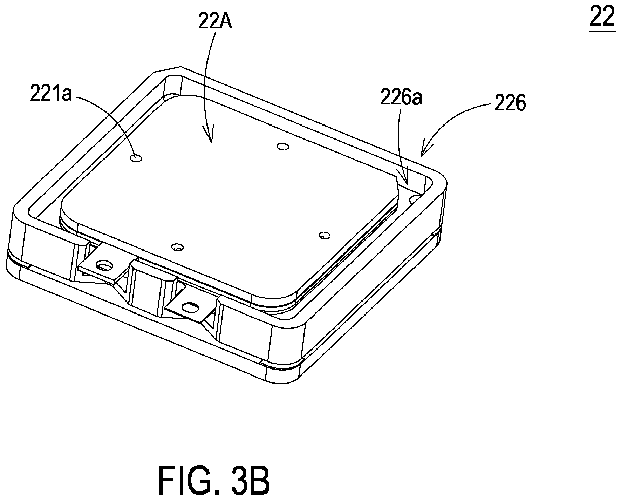

The band structure 20 of the wearable device 2 can be not only used for surrounding and fitting the specific part of the user, but also be used for supporting the main body 21. As mentioned in the above paragraphs, the main body 21 is connected to and disposed on the outer surface 200 of the band structure 20. The main body 21 may be integrally formed with the band structure 20 or may be fixed on the band structure 20 by a buckle structure, but not limited herein. In this embodiment, the main body 21 is a square and hollowed frame structure, the shape of which is substantially smaller or equal to the width of the band structure 20, but the shape and size of the main body 21 are not limited to and can be varied to meet the practical demands. The receiving space of the main body 21 is mainly provided for the micro air pump 22 (as shown in FIG. 3B) and the driving control module 25 to be accommodated therein.

Please refer to FIG. 2. In this embodiment, the wearable device 2 further comprises a display panel 27 disposed upon the micro air pump 22. The display panel 27 may be a touch control screen or a screen with buttons, through which the user is able to select the sensor or the optical sensor to sense the physiological data. The display panel 27 displays information which includes but not limited to the physiological data of the user, time data and coming call data. In some other embodiments, the micro air pump 22 of the wearable device 2 is covered by a cover body (not shown), which is not limited to the display panel 27.

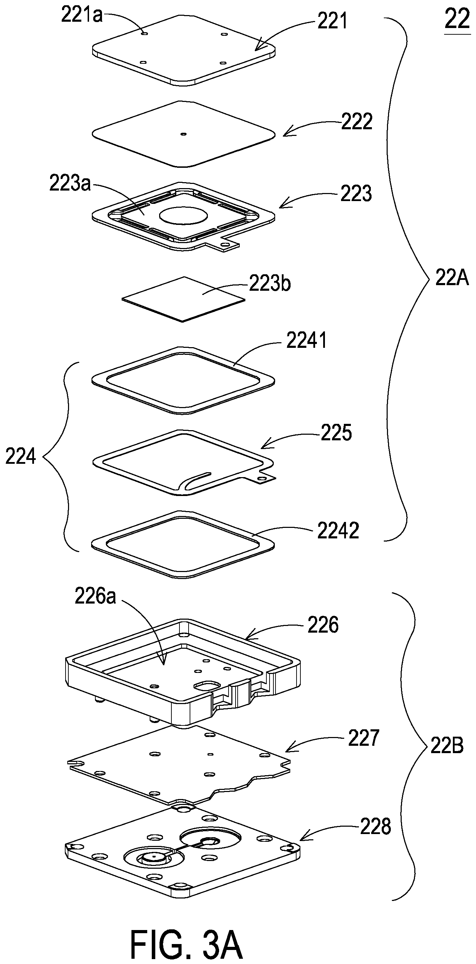

Please refer to FIG. 3A and FIG. 3B. FIG. 3A schematically illustrates the exploded view of a micro air pump of the wearable device shown in FIG. 2. FIG. 3B schematically illustrates the assembled structure of the micro air pump shown in FIG. 3A. In this embodiment, the micro air pump 22 is a micro pneumatic power device that is piezoelectrically actuated, but not limited herein. The micro pneumatic power device (i.e. the micro air pump 22) comprises a micro air transportation device 22A and a micro valve device 22B. When gas is transported from the micro air transportation device 22A into the micro valve device 22B, an operation of pressure collection or pressure relief is selectively performed. The micro air transportation device 22A comprises an air inlet plate 221, a resonance plate 222, a piezoelectric actuator 223, an insulated plate 224 and a conductive plate 225. The piezoelectric actuator 223 is disposed corresponding to the resonance plate 222, and the air inlet plate 221, the resonance 222, the piezoelectric actuator 223, the insulated plate 2241, the conductive plate 225 and the insulated plate 2242 are sequentially stacked. The piezoelectric actuator 223 is assembled by a float plate 223a and a piezoelectric ceramic plate 223b. The micro valve device 22B comprises an air collection plate 226, a valve plate 227 and an outlet plate 228 that are sequentially stacked. The air collection plate 226 can be a single plate or a frame structure with peripheral sidewalls as shown in FIG. 3A, wherein the peripheral sidewalls are protruding from the bottom edges of a bottom plate of the frame structure and an accommodation space 226a is defined by both the peripheral sidewalls and the bottom plate of the frame structure. After assembling the micro pneumatic power device 22, the front view is shown in FIG. 3B. That is, the micro air transportation device 22A is accommodated within the accommodation space 226a of the air collection plate 226 and stacking on the valve plate 227 and the outlet plate 228. Through the assembly of the air transportation device 22A and the micro valve device 22B, the piezoelectric actuator 223 is driven to make the gas introduced from at least an air inlet hole 221a of the air inlet plate 221 of the micro air transportation device 22A, flowing through a plurality of pressure chambers (not shown) and transported downwardly. Moreover, the gas flows in one direction inside the micro valve device 22B by which pressure is accumulated in an airbag 23 (as shown in FIC. 4B) connected to an outlet end of the micro valve device 22B for performing the operation of pressure collection. Otherwise, the operation of pressure relief is performed by adjusting an outlet amount of the micro air transportation device 22A and making the gas discharged through a pressure relief hole (not shown) on the outlet plate 228 of the micro valve device 22B for releasing pressure.

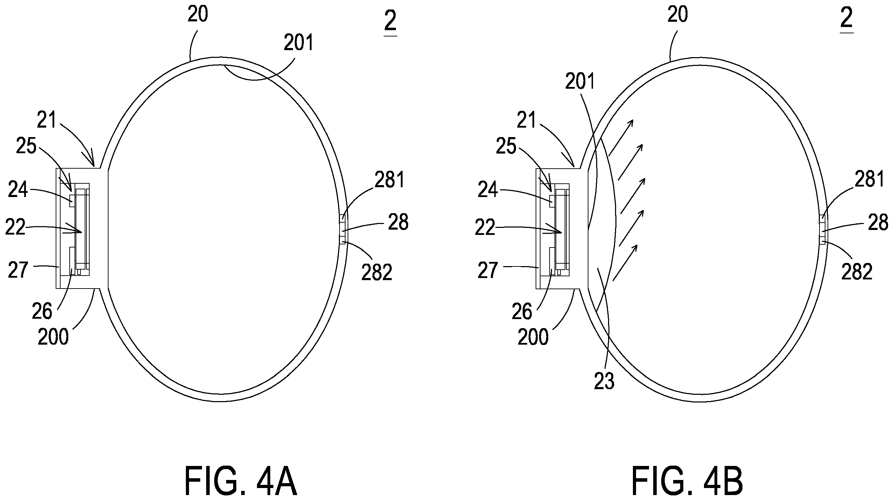

Please refer to FIG. 4A and FIG. 4B. FIG. 4A schematically illustrates a side view of the wearable device shown in FIG. 2. FIG. 4B schematically illustrates an inflated state of the airbag of a wearable device according to an embodiment of the present disclosure. The band structure 20 may further have a receiving portion (not shown) formed on the inner surface 201 opposing to the main body 21 for receiving the airbag 23. The receiving portion is communicated with the receiving space of the main body 21, but not limited thereto. When the micro air pump 22 is not operated, the side view of the wearable device 2 is shown in FIG. 4A. As shown, only the band structure 20 and the main body 21 can be seen and the airbag 23 cannot be seen because the airbag 23 is received by the receiving portion. However, when the micro air pump 22 is driven to operate, the gas will be transported to the airbag 23 through the outlet end of the micro air pump 22, so that the airbag 23 is inflated and expended outwardly as shown in FIG. 4B.

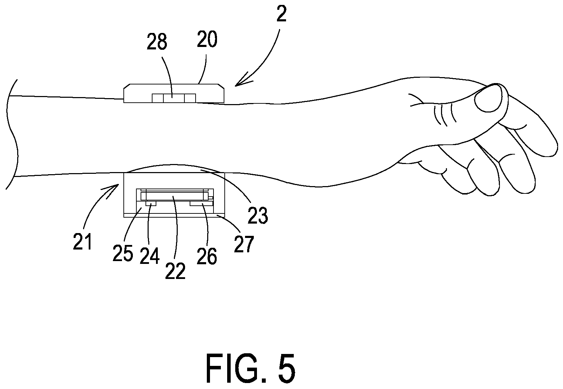

Please refer to FIG. 5. FIG. 5 schematically illustrates a wearable device according to an embodiment of the present disclosure worn on a wrist of a user. When the wearable device 2 of the present disclosure is worn on the wrist of the user and the driving control module 25 drives the micro air pump 22 to operate, the micro pump 22 transports gas from the outlet end thereof into the airbag 23, so that the airbag is inflated and expended to be fixed on the inner side of the wrist of the user. At this time, the user is allowed to select the sensor 24, which is disposed on the driving control module 25, to directly sense the atmospheric change of the micro air pump 22 for calculating the physiological data which is then recorded in the memory 241 of the driving control module 25, by which a precise detection is achieved. Otherwise, the user is also allowed to select the optical sensor 28 to detect in which the light emitter 281 of the optical sensor 28 emits a sensing light to the skin of the wrist of the user for sensing the physiological data of the user, and the light receiver 282 of the optical sensor 28 receives the sensing light beam. Afterwards, the physiological data is recorded in the memory 241 of the driving control module 25, and a rapid detection is achieved. In this embodiment, the physiological data includes but not limited to the pulse, the blood pressure and the heart-beating rate.

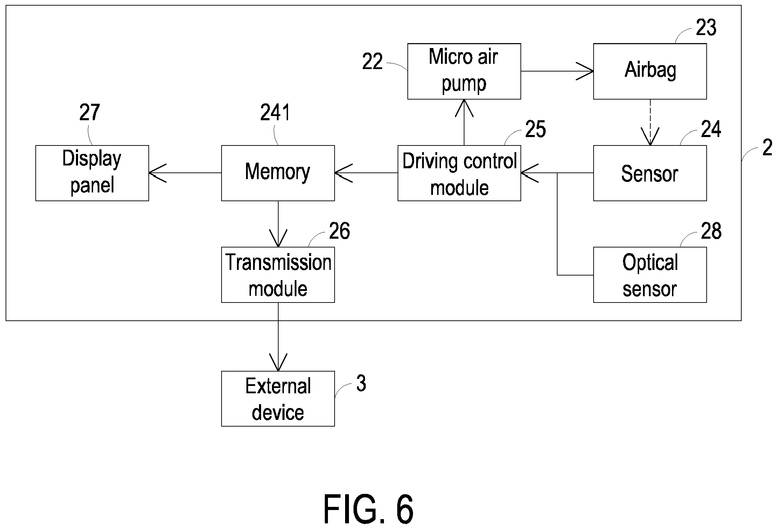

Please refer to FIG. 6. In this embodiment, the wearable device 2 may further comprise a transmission module 26. The transmission module 26 is disposed on the driving control module 25 and is for transmitting the physiological data, which is measured by the ways mentioned above, to an external device 3 for further analysis. Thus, the user is able to realize more detailed health information. The position on which the transmission module 26 is disposed is not limited herein. In some embodiments, the transmission module 26 can be a wired transmission module (e.g. a USB, a mini-USB or a micro-USB). In some other embodiments, the transmission module 26 can be a wireless transmission module (e.g. a Wi-Fi module, a Bluetooth module, a RFID module or a NFC module). The transmission module 26 may further comprise at least a wired transmission module and at least a wireless transmission module. The data transmission type can be varied to meet the practical demands. All manners that may store the physiological data of the user in the memory 241 of the driving control module 25 are covered in the protection scope of the present disclosure and are not redundantly described herein. In this embodiment, the external device 3 includes but not limited to at least one of a cloud system, a portable device and a computer system. The external device 3 is capable of receiving the physiological data transmitted by the wearable device 2 of the present disclosure and then analyzes and compares the physiological data, so that the health status of the user can be much more realized.

Please refer to FIG. 2 and FIG. 6. When a precise detection is performed by the wearable device 2 of the present disclosure, the driving control module 25 drives the micro air pump 22 to operate, thereby transporting gas into the airbag 23 to inflate the airbag 23, and the inflated and expended airbag 23 presses upon a specific part of the user. Meanwhile, the physiological data of the user is sensed by the sensor 24. When the physiological data of the user is sensed through the sensor 24 and recorded, the precise detection is achieved. In addition, when a rapid detection is performed by the wearable device 2, the physiological data of the user is mainly sensed through operation of the optical sensor 28, and the physiological data is transmitted to driving control module 25 for being recorded. In some embodiments, the physiological data is directly displayed on the display panel 27. In some embodiments, the physiological data is transmitted to an external device 3 by the transmission module 26 for further analysis.

From the above description, the wearable device of the present disclosure provides selection of the detecting ways for the user that the user is allowed to select a precise detection of the physiological data by using the sensor, in which the piezoelectrically actuated micro air pump transports gas into the airbag then the sensor performs detection of the physiological data of the user. The user is also allowed to select a rapid detection of the physiological data by using the optical sensor, in which the optical sensor detects the physiological data by the light emitter and the light receiver thereof. The physiological data is finally sent to the memory of the driving control module and is selectively transmitted to the external devices and selectively displayed on the display panel of the wearable device. As so, a precise detection or a rapid detection is achieved. Advantageously, the wearable device of the present disclosure is small, light, easily to carry and power-saving, achieving a significant improvement in the field.

## Claims

1. A wearable device (2), comprising:
a band structure (20) having an outer surface (200) and an inner surface (201);
a main body (21) disposed on the outer surface (200) of the band structure (20) and connected thereto, the main body (21) has a receiving space;
a micro air pump (22) disposed within the receiving space of the main body (21);
an airbag (23) disposed on the inner surface (201) of the band structure (20) and opposing to the micro air pump (22), the airbag (23) is communicated with the micro air pump (22);
a driving control module (25) disposed within the receiving space of the main body (21);
a sensor (24) disposed on the driving control module (25) and connected with the micro air pump (22); and
an optical sensor (28) disposed on the inner surface (201) of the band structure (20) and electrically connected with the driving control module (25),
wherein the driving control module (25) controls the micro air pump (22) to operate, thereby transporting gas into the airbag (23) from the micro air pump (22) to make the airbag (23) inflated and expended to be fixed on a specific part of the user, wherein the physiological data of the user is selectively sensed by the sensor (24) and transmitted to the driving control module (25) to be recorded by which a precise detection is achieved, and wherein the physiological data of the user is selectively sensed by the optical sensor (28) and transmitted to the driving control module (25) to be recorded by which a rapid detection is achieved.

2. The wearable device (2) according to claim 1, wherein the micro air pump (22) is a micro pneumatic power device which is piezoelectrically actuated, and the micro pneumatic power device comprises a micro air transportation device (22A) and a micro valve device (22B), wherein when gas is transported from the micro air transportation device (22A) into the micro valve device (22B), an operation of pressure collection or an operation of pressure relief is selectively performed.

3. The wearable device (2) according to claim 2, wherein the micro air transportation device (22A) comprises an air inlet plate (221), a resonance plate (222) and a piezoelectric actuator (223) which are sequentially stacked, wherein when the piezoelectric actuator (223) is driven, the gas is entered from the air inlet plate (221), flowing through a plurality of pressure chambers and being transported downwardly to flow in one direction within the micro valve device (22B), wherein the micro valve device (22B) comprises an air collection plate (226), a valve plate (227) and an outlet plate (228) which are sequentially stacked, an outlet end of the outlet plate (228) is in air communication with the airbag (23), wherein when gas is transported from the micro air transportation device (22A) into the micro valve device (22B), the operation of pressure collection is selectively performed by transporting gas to the airbag (23) through the outlet end of the outlet plate (228), or the operation of pressure relief is selectively performed through a pressure relief hole of the outlet plate (228).

4. The wearable device (2) according to claim 1 further comprising a transmission module (26) disposed on the driving control module (25) and transmitting the physiological data of the user to an external device (3).

5. The wearable device (2) according to claim 4, wherein the transmission module (26) is at least one of a wired transmission module and a wireless transmission module.

6. The wearable device (2) according to claim 5, wherein the wired transmission module is at least one of a USB, a mini-USB and a micro-USB, and the wireless transmission module is at least one of a Wi-Fi module, a Bluetooth module, a RFID module and a NFC module.

7. The wearable device (2) according to claim 4, wherein the external device (3) is at least one of a cloud system, a portable device and a computer system.

8. The wearable device (2) according to claim 1, wherein the optical sensor (28) has a light emitter (281) and a light receiver (282), the light emitter (281) emits a sensing light beam for sensing the physiological data of the user, and then the light receiver (282) receives the sensing light beam.

9. The wearable device (2) according to claim 1 further comprising a display panel (27) configured to display an information.

10. The wearable device (2) according to claim 9, wherein the information comprises at least one of the physiological data of the user, time data and coming call data.

11. The wearable device (2) according to claim 9, wherein the display panel (27) is a touch control screen or a screen with buttons, and the sensor (24) or the optical sensor (28) is selected through the touch control screen by the user to sense the physiological data.

12. The wearable device (2) according to claim 1, wherein the driving control module (25) further comprises a memory for storing the physiological data.
